**Europäisches Patentamt**

**European Patent Office**

**Office européen des brevets**

(19)

(11) Veröffentlichungsnummer : **0 074 488**
**B1**

(12)

# EUROPÄISCHE PATENTSCHRIFT

(45) Veröffentlichungstag der Patentschrift :
03.04.85

(21) Anmeldenummer : 82107086.9

(22) Anmeldetag : 05.08.82

(51) Int. Cl.⁴ : **C 07 C117/08, C 07 C 99/00,
C 07 C101/30**

(54) 2-Azido-3-benzyloxy-propionsäure-benzylester, Verfahren zu dessen Herstellung und dessen Verwendung.

(30) Priorität : 10.09.81 DE 3135840

(43) Veröffentlichungstag der Anmeldung :
23.03.83 Patentblatt 83/12

(45) Bekanntmachung des Hinweises auf die Patenterteilung : 03.04.85 Patentblatt 85/14

(84) Benannte Vertragsstaaten :
AT BE CH DE FR GB IT LI NL

(56) Entgegenhaltungen :
US-A- 3 471 523
METHODEN DER ORGANISCHEN CHEMIE (HOUBEN-
WEYL), Band XI/1, 1957 GEORG THIEME VERLAG,
Stuttgart

(73) Patentinhaber : Degussa Aktiengesellschaft
Weissfrauenstrasse 9
D-6000 Frankfurt am Main 1 (DE)

(72) Erfinder : Effenberger, Franz, Dr. Prof.
Schatzweg 5
D-7000 Stuttgart 70 (DE)
Erfinder : Zoller, Gerhard, Dr. Dipl.-Chem.
Langwiese 16
D-6457 Maintal 2 (DE)

**Beschreibung**

Gegenstand der Erfindung ist der 2-Azido-3-benzyloxy-propionsäure-benzylester und ein Verfahren zu dessen Herstellung, welches dadurch gekennzeichnet ist, dass man 2-Chlor-acrylnitril mit der zweifachen molaren Menge Benzylalkohol zum 3-Benzyloxy-2-chlor-propionsäure-iminobenzylester umsetzt, diesen sauer zum 3-Benzyloxy-2-chlor-propionsäure-benzylester verseift und schliesslich das Chloratom mittels eines Alkalimetallazids in Gegenwart eines Phasentransferkatalysators gegen eine Azidogruppe austauscht.

Der neue 2-Azido-3-benzyloxy-propionsäure-benzylester kann der katalytischen Hydrierung unterworfen werden, wobei die Azidogruppe zu einer Aminogruppe reduziert wird. Je nach den angewandten Reaktionsbedingungen können dabei direkt wahlweise z. B. D,L-Serin, O-Benzyl-D,L-serin, O-Benzyl-D,L-serin-benzylester oder ein Alkylester des D,L-Serins oder die entsprechenden Hydrochloride erhalten werden.

Die Reduktion von Azidoverbindungen zu entsprechenden Aminen ist aus Houben-Weyl, Methoden der organischen Chemie, Band XI/1, 1957, Seiten 539 ff., grundsätzlich bekannt.

Aus J. biol. Chem. *105*, Seite 555 (1934), ist ferner eine Reaktionsfolge bekannt, bei der α-Brom-β-hydroxy-propionsäure-methylester mit Natriumazid umgesetzt und die erhaltene Azidoverbindung katalytisch reduziert wird. Durch diese Reaktionsfolge wird jedoch nicht Serin, sondern vielmehr Isoserin erhalten.

Der 2-Azido-3-benzyloxy-propionsäure-benzylester ist demnach eine wertvolle Schlüsselsubstanz für die Herstellung von D,L-Serin und Derivaten des D,L-Serins.

Ein weiterer Gegenstand der Erfindung ist daher die Verwendung des 2-Azido-3-benzyloxy-propionsäure-benzylesters als Zwischenprodukt bei der Herstellung von D,L-Serin oder Derivaten des D,L-Serins.

Das erfindungsgemäße Verfahren zur Herstellung des 2-Azido-3-benzyloxy-propionsäure-benzylesters geht aus von dem wohlfeilen, stabilen und technisch gut zu handhabenden 2-Chlor-acrylnitril und verläuft in allen Reaktionsstufen mit hohen Ausbeuten. Aber auch bei der Weiterverarbeitung zu D,L-Serin oder Derivaten des D,L-Serins werden hohe Ausbeuten erzielt.

In der ersten Reaktionsstufe wird das 2-Chlor-acrylnitril mit der mindestens zweifachen molaren Menge Benzylalkohol umgesetzt. Besonders vorteilhaft ist es, den Benzylalkohol im Überschuß, insbesondere in einer Menge von 2,1 bis 10 Mol pro Mol eingesetztem 2-Chlor-acrylnitril, anzuwenden. Die Umsetzung erfolgt unter dem katalytischen Einfluß eines Alkalimetallbenzylats, das als solches zugesetzt oder in situ aus einem Alkalimetall und dem Benzylalkohol gebildet werden kann. Besonders bevorzugt wird die Verwendung von Natriumbenzylat. Das Alkalimetallbenzylat wird zweckmäßig in einer Menge von 0,5 bis 50 Molprozent, bezogen auf das eingesetzte 2-Chlor-acrylnitril, eingesetzt, vorzugsweise in einer Menge von etwa 5 Molprozent.

Der zweckmässige Temperaturbereich für die Umsetzung liegt zwischen − 35 und + 80 °C, insbesondere zwischen 0 und + 5 °C. In dieser ersten Reaktionsstufe wird das 2-Chlor-acrylnitril in den 3-Benzyloxy-2-chlor-propionsäure-iminobenzylester umgewandelt, der nach Neutralisation des rohen Reaktionsgemisches mi' Kohlendioxid durch fraktionierte Destillation bei vermindertem Druck isoliert werden kann. Seine Isolierung ist jedoch nicht unbedingt erforderlich, es kann vielmehr auch das rohe Reaktionsgemisch unmittelbar weiterverarbeitet werden.

In der zweiten Reaktionsstufe wird nun der rohe oder durch Destillation gereinigte 3-Benzyloxy-2-chlor-propionsäure-iminobenzylester in Gegenwart einer Mineralsäure zum 3-Benzyloxy-2-chlor-propionsäure-benzylester verseift. Ein besonders zweckmässiges Verseifungsmittel ist etwa 10 gewichtsprozentige wässrige Salzsäure. Die Verseifung erfolgt zweckmässig bei Raumtemperatur und ist im allgemeinen innerhalb längstens einer Stunde vollständig. Der gebildete 3-Benzyloxy-2-chlor-propionsäure-benzylester wird mit Diethylether aus dem rohen Verseifungsgemisch extrahiert und nach Trocknung des Extrakts, beispielsweise mit Magnesiumsulfat, durch fraktionierte Destillation bei vermindertem Druck in reiner Form gewonnen.

In einer dritten Reaktionsstufe wird schliesslich das Chloratom des 3-Benzyloxy-2-chlor-propionsäure-benzylesters mittels eines Alkalimetallazids gegen eine Azidogruppe ausgetauscht. Das Alkalimetallazid, vorzugsweise Natriumazid, wird in Form einer wässrigen Lösung eingesetzt. Da die Umsetzung zur Vermeidung von unerwünschten Nebenreaktionen zweckmässigerweise bei einer verhältnismässig niedrigen Temperatur im Bereich zwischen Raumtemperatur und 100 °C, beispielsweise bei etwa 60 °C, vorgenommen wird, ist die Gegenwart eines Phasentransferkatalysators erforderlich. Geeignet sind alle literaturbekannten Phasentransferkatalysatoren, wie quaternäre Ammonium- und Phosphoniumsalze oder Kronenether. Bevorzugt werden quaternäre Ammoniumsalze, insbesondere ein handelsübliches Tri-($C_8$ bis $C_{10}$-alkyl)-methyl-ammoniumchlorid (« Tricaprylylmethylammoniumchlorid » ; Aliquat 336), verwendet. Der Phasentransferkatalysator wird zweckmässigerweise in einer Menge von etwa 5 Molprozent, bezogen auf den eingesetzten 3-Benzyloxy-2-chlor-propionsäure-benzylester, angewandt. Das Alkalimetallazid wird zweckmässigerweise im Überschuss, beispielsweise im 1,1- bis 1,5-fachen der theoretisch erforderlichen Menge, eingesetzt. Der Austausch des Chloratoms gegen die Azidogruppe erfordert im allgemeinen eine Reaktionszeit von 8 bis 10 Stunden. Die Isolierung des gebildeten 2-Azido-3-benzyloxy-

2

propionsäure-benzylesters erfolgt vorteilhaft so, dass man das rohe Reaktionsgemisch mehrmals mit Methylenchlorid extrahiert, die vereinigten Extrakte, beispielsweise mit Magnesiumsulfat, trocknet und eine chromatographische Trennung in einer Kieselgelsäule vornimmt. Als Laufmittel für die Trennung ist ein Gemisch aus 15 Volumteilen Petrolether und 1 Volumteil Essigsäure-ethylester besonders geeignet.

Bei dieser chromatographischen Trennung werden überraschenderweise alle Verunreinigungen bzw. Nebenprodukte in der Kieselgelsäule so festgehalten, dass das Eluat nach dem Eindampfen, zweckmässigerweise bei vermindertem Druck, ein farbloses Öl ergibt, das nach seinen elementaranalytischen und spektroskopischen Daten aus reinem 2-Azido-3-benzyloxy-propionsäure-benzylester besteht.

Diese neue Verbindung dient als wertvolles Zwischenprodukt für die Herstellung von D,L-Serin, D,L-Serin-hydrochlorid, D,L-Serin-methylester-hydrochlorid, D,L-Serinethylester-hydrochlorid, O-Benzyl-D,L-Serin-benzylesterhydrochlorid oder O-Benzyl-D,L-serin.

Zur Herstellung von D,L-Serin wird der 2-Azido-3-benzyloxy-propionsäure-benzylester in einem inerten Lösungsmittel, beispielsweise Aceton, gelöst und mit trockenem Salzsäuregas versetzt. Das Salzsäuregas wird in der mindestens äquimolaren Menge eingesetzt, es kann aber auch in einem Überschuss bis etwa 10 : 1 verwendet werden.

Dann wird nach Zusatz eines Palladium-Katalysators in einem Autoklaven bis zum vollständigen Umsatz hydriert. Als Palladium-Katalysatoren können z. B. des feinverteilte Palladiummohr oder Palladiumverbindungen, wie Palladiumchlorid-, -bromid, -jodid, -nitrat, -oxid oder -oxidhydrat, oder Palladium-Komplexsalze, wie Tetrachloropalladate oder Hexachloropalladate, eingesetzt werden. Besonders bevorzugt wird die Verwendung des Palladium-Katalysators in Form eines Trägerkatalysators. Geeignete Trägermaterialien sind beispielsweise Kieselgele, Aluminiumoxide, Zeolithe, Bariumsulfat oder Calciumcarbonat, vorzugsweise aber Aktivkohle.

Der Palladium-Katalysator wird zweckmässigerweise in einer Menge zwischen, 0,001 und 5 Gewichtsprozent, berechnet als aktives Metall und bezogen auf das Gewicht des eingesetzten 2-Azido-3-benzyloxy-propionsäure-benzylesters, vorzugsweise in einer Menge zwischen 0,1 und 1,0 Gewichtsprozent, eingesetzt. Der Wasserstoffdruck bei der Hydrierung kann zwischen 1 und 100 bar gewählt werden, vorzugsweise zwischen 1 und 80 bar. Die Hydrierung kann bei einer Temperatur zwischen 0 und 100 °C, vorzugsweise zwischen 20 und 80 °C, vorgenommen werden.

Nach beendeter Wasserstoffaufnahme wird der Katalysator zusammen mit dem gebildeten D,L-Serin-hydrochlorid abfiltriert und das Filtrat verworfen. Der Filterrückstand wird mit heissem Wasser extrahiert und der Extrakt, beispielsweise in einem Rotationsverdampfer, eingeengt. Der erhaltene Rückstand wird anschliessend mit Ammoniak neutralisiert. Nach Zugabe von Ethanol und Abkühlen fällt reines D,L-Serin aus, das abfiltriert und unter vermindertem Druck, zweckmässigerweise bei einer Temperatur zwischen 25 und 60 °C, getrocknet werden kann.

Alternativ kann zur Herstellung von D,L-Serin auch so verfahren werden, dass der 2-Azido-3-benzyloxy-propionsäure-benzylester in einem inerten Lösungsmittel, beispielsweise Ethanol, gelöst wird und ohne Zusatz von Salzsäuregas zunächst bei einer Temperatur zwischen 10 und 30 °C bei einem Wasserstoffdruck zwischen 30 und 50 bar in der angegebenen Weise mit dem Palladium-Katalysator hydriert wird. Danach wird mit Wasser verdünnt, eine weitere Portion des Palladium-Katalysators zugegeben und bei einer Temperatur zwischen 60 und 80 °C und einem Wasserstoffdruck zwischen 40 und 70 bar bis zum vollständigen Umsatz weiterhydriert. Die Wassermenge soll zweckmässigerweise etwa dem halben Volumen des verwendeten Ethanols, die zusätzliche Katalysator-Portion etwa der Hälfte der ursprünglich eingesetzten Menge entsprechen. Nach beendeter Wasserstoffaufnahme wird der Katalysator abfiltriert, das Filtrat eingedampft und das als fester Rückstand erhaltene D,L-Serin bei vermindertem Druck und einer Temperatur von etwa 50 °C getrocknet.

Zur Herstellung von D,L-Serin-hydrochlorid wird der 2-Azido-3-benzyloxy-propionsäure-benzylester, wie oben beschrieben, nach Zugabe von Salzsäuregas hydriert. Nach beendeter Wasserstoffaufnahme wird der Katalysator zusammen mit dem gebildeten D,L-Serin-hydrochlorid abfiltriert und das Filtrat verworfen. Der Filterrückstand wird mit heissem Wasser extrahiert und der Extrakt eingedampft. Der verbleibende ölige Rückstand wird durch Zusatz von Diethylether zur Kristallisation gebracht. Die aus reinem D,L-Serin-hydrochlorid bestehenden Kristalle werden abgesaugt und bei vermindertem Druck und einer Temperatur von etwa 50 °C getrocknet.

Zur Herstellung von D,L-Serin-alkylester-hydrochloriden wird so verfahren, dass der 2-Azido-3-benzyloxy-propionsäure-benzylester in dem jeweiligen Alkanol, z. B. Methanol oder Ethanol, gelöst und mit einem Überschuss an Salzsäuregas versetzt wird. Dann wird wieder, wie oben beschrieben, in Gegenwart eines Palladium-Katalysators hydriert, aber zweckmässigerweise bei einer Temperatur zwischen 20 und 30 °C und einem Wasserstoffdruck zwischen 10 und 30 bar.

Nach beendeter Wasserstoffaufnahme wird der Katalysator abfiltriert, das Filtrat eingeengt und der verbleibende Rückstand aus einem Gemisch von etwa gleichen Volumenteilen-Alkanol und Diethylether umkristallisiert. Man erhält so die reinen D,L-Serin-alkylester-hydrochloride.

O-Benzyl-D,L-serin-benzylester-hydrochlorid lässt sich aus dem 2-Azido-3-benzyloxy-propionsäure-benzylester dadurch erhalten, dass man die Hydrierung in Ethanol als Lösungsmittel und nach Zusatz der mindestens äquimolaren Menge an Salzsäuregas vornimmt, aber anstelle eines Palladium-Katalysators Rhenium-(VII)-sulfid in einer Menge zwischen 0,001 und 20 Gewichtsprozent, bezogen auf das Gewicht des eingesetzten 2-Azido-3-benzyloxypropionsäure-benzylesters, als Katalysator einsetzt. Die Reaktionstemperatur soll zweckmässigerweise wieder zwischen 20 und 30 °C, der Wasserstoffdruck zwischen 10 und 30 bar liegen.

Nach beendeter Wasserstoffaufnahme wird der Katalysator abfiltriert und das Filtrat eingedampft. Der verbleibende Rückstand wird in Chloroform aufgenommen und das O-Benzyl-D,L-serin-benzylester-hydrochlorid durch Zugabe von Diethylether ausgefällt. Zur weiteren Reinigung kann es aus einem Gemisch von Ethanol und Diethylether umkristallisiert werden.

O-Benzyl-D,L-serin kann so erhalten werden, dass man den 2-Azido-3-benzyloxy-propionsäure-benzylester in Ethanol als Lösungsmittel und in Gegenwart eines Palladium-Katalysators in den oben angegebenen Mengen drucklos oder bei einem Wasserstoffdruck bis zu 40 bar bei einer Temperatur zwischen 10 und 30 °C hydriert.

Nach beendeter Wasserstoffaufnahme wird filtriert und das Filtrat verworfen. Der Filterrückstand wird mit heissem Wasser extrahiert und der Extrakt unter vermindertem Druck eingedampft. Das ausfallende O-Benzyl-D,L-serin wird abgesaugt und unter vermindertem Druck bei etwa 50 °C getrocknet.

Die Herstellung des 2-Azido-3-benzyloxy-propionsäurebenzylesters und seine Weiterverarbeitung zu D,L-Serin bzw. zu verschiedenen Derivaten des D,L-Serins wird durch die nachstehenden Beispiele näher erläutert. Prozentangaben bedeuten, sofern nicht anders angegeben, Gewichtsprozente.

### Beispiel 1

0,23 g (0,01 Mol) Natrium werden in 108,14 g (1,0 Mol) Benzylalkohol gelöst. Zu dieser frisch bereiteten Natriumbenzylatlösung in Benzylalkohol werden 17,50 g (0,20 Mol) 2-Chloracrylnitril bei 0 °C langsam zugetropft. Nach Beendigung des Zutropfens wird 1 Stunde bei 0 °C und weitere 2 Stunden bei Raumtemperatur nachgerührt. Das Reaktionsgemisch wird mit 60 ml Diethylether versetzt und dann werden unter kräftigem Rühren 60 ml einer 10 %igen Salzsäure zugetropft. Anschliessend wird noch eine weitere Stunde bei Raumtemperatur gerührt. Die wässrige Phase wird dreimal mit je 50 ml Diethylether extrahiert und die vereinigten organischen Phasen mit Magnesiumsulfat getrocknet. Nach Abdampfen des Lösungsmittels erhält man in 87 %iger Ausbeute den rohen 3-Benzyloxy-2-chlor-propionsäure-benzylester. Nach Fraktionierung im Vakuum erhält man 43,9 g (72 % der Theorie) reinen 3-Benzyloxy-2-chlor-propionsäurebenzylester mit einem Siedepunkt von 153-155 °C bei 0,006 5 mbar.

$\eta_D^{20}$ : 1,547 2
IR (flüssig kapillar) : 1 740 (C = O) cm$^{-1}$
$C_{17}H_{17}ClO_3$ (304,776)
Berechnet : C 67,00 % H 5,62 % Cl 11,63 %
Gefunden : C 67,19 % H 5,67 % Cl 11,37 %

### Beispiel 2

a) Darstellung von 3-Benzyloxy-2-chlor-propionsäureiminobenzylester

0,23 g (0,01 Mol) Natrium werden in 108,14 g (1,0 Mol) Benzylalkohol gelöst. Zu dieser frisch bereiteten Natriumbenzylatlösung in Benzylalkohol werden bei 5 °C langsam 17,50 g (0,20 Mol) 2-Chloracrylnitril zugetropft. Danach wird 1 Stunde bei 5 °C und weitere 2 Stunden bei Raumtemperatur nachgerührt. In die Reaktionslösung wird Kohlendioxid eingeleitet, der ausgefallene Feststoff abfiltriert und das Filtrat im Vakuum fraktioniert. Bei 150-154 °C und einem Druck von 0,006 5 mbar gehen 48,6 g reiner 3-Benzyloxy-2-chlor-propionsäure-iminobenzylester über.

$\eta_D^{20}$ : 1,556 9
IR (flüssig kapillar) : 1 660 (C = N) ; 3 315 (N-H) cm$^{-1}$
$C_{17}H_{18}ClNO_2$ (303,79)
Berechnet : C 67,21 % H 5,97 % Cl 11,67 % N 4,61 %
Gefunden : C 67,47 % H 6,04 % Cl 11,44 % N 4,81 %

b) Verseifung des nach a) erhaltenen 3-Benzyloxy-2-chlorpropionsäure-iminobenzylesters

15,19 g (0,05 Mol) 3-Benzyloxy-2-chlor-propionsäureiminobenzylester werden in 30 ml 10 %iger Salzsäure 1 Stunde bei 25 °C gerührt. Danach wird das Reaktionsgemisch zweimal mit je 20 ml Diethylether extrahiert, mit einer Natriumhydrogencarbonatlösung neutral gewaschen und die Etherphase über Magnesiumsulfat getrocknet. Nach Abdampfen des Ethers wird im Vakuum fraktioniert.

Man erhält 13,1 g (86 % der Theorie) reinen 3-Benzyloxy-2-chlor-propionsäure-benzylester mit einem Siedepunkt von 153-155 °C bei 0,006 5 mbar.

### Beispiel 3

4,57 g (0,015 Mol) des nach Beispiel 1 oder 2 erhaltenen 3-Benzyloxy-2-chlor-propionsäure- benzylesters werden zu einer Lösung von 1,46 g (0,022 5 Mol) Natriumazid in 6 ml Wasser gegeben. Nach Zugabe von 0,31 g (5 Molprozent) Aliquat 336 als Phasentransferkatalysator wird das Gemisch unter intensivem Rühren 9 Stunden bei 60 °C gehalten. Es wird dreimal mit je 15 ml Methylenchlorid extrahiert, die vereinigten Extrakte werden über Magnesiumsulfat getrocknet, das Methylenchlorid wird abgedampft und der Rückstand über eine 40 cm-Kieselgelsäule mit dem Laufmittel Petrolether/Essigsäureethylester (Volumenverhältnis 15 : 1) gereinigt.

Nach Eindampfen des Eluats unter vermindertem Druck erhält man 3,83 g (82 % der Theorie) 2-Azido-3-benzyloxy-propionsäure-benzylester als farbloses Öl.

$\eta_D^{20}$ : 1,545 8
IR (flüssig kapillar) : 1 745 (C = O) ; 2 100 (Azid) cm$^{-1}$
$C_{17}H_{17}N_3O_3$ (311,34)
Berechnet :   C 65,58 %   H 5,50 %   N 13,50 %
Gefunden :   C 65,39 %   H 5,60 %   N 13,60 %

## Beispiel 4

1,56 g (0,005 Mol) 2-Azido-3-benzyloxy-propionsäurebenzylester werden in 10 ml absolutem Aceton gelöst, 0,90 g (0,025 Mol) trockenes Salzsäuregas eingeleitet und zu dieser Lösung 0,20 g Palladium auf Aktivkohle (10 %ig) zugesetzt. Diese Reaktionsmischung wird in einem Autoklaven 20 Stunden bei einem Wasserstoffdruck von 20 bar und einer Reaktionstemperatur von 25 °C hydriert. Nach Filtration wird der Filterrückstand mit heissem Wasser extrahiert, der Extrakt am Rotationsverdampfer eingedampft und das zurückbleibende Öl durch Zugabe von Diethylether zur Kristallisation gebracht. Die Kristalle werden abgesaugt und 24 Stunden bei 50 °C unter vermindertem Druck getrocknet.

Man erhält so 0,57 g (81 % der Theorie) D,L-Serin-hydrochlorid mit einem Schmelzpunkt von 140-145 °C (Literatur : 140-142 °C).

Die Elementaranalyse ergibt übereinstimmende Werte :

$C_3H_8ClNO_3$ (141,56)
Berechnet :   C 25,46 %   H 5,70 %   Cl 25,05 %   N 9,89 %
Gefunden :   C 25,48 %   H 5,56 %   Cl 24,87 %   N 9,76 %

Das D,L-Serin-hydrochlorid ist dünnschichtchromatographisch rein.

## Beispiel 5

1,56 g (0,005 Mol) 2-Azido-3-benzyloxy-propionsäure-benzylester werden in 10 ml absolutem Aceton gelöst, 0,30 g (0,008 Mol) trockenes Salzsäuregas eingeleitet und 0,20 g Palladium auf Aktivkohle (10 %ig) zugesetzt. Es wird anschliessend in einem Autoklaven 20 Stunden bei einem Wasserstoffdruck von 20 bar und einer Reaktionstemperatur von 25 °C hydriert. Nach Filtration wird der Filterrückstand mit heissem Wasser extrahiert, der Extrakt am Rotationsverdampfer eingedampft und der ölige Rückstand mit Ammoniak neutralisiert. Nach Zugabe von 25 ml Ethanol wird auf 0 °C abgekühlt. Das so ausgefällte D,L-Serin wird abgesaugt und 24 Stunden bei 50 °C unter vermindertem Druck getrocknet.

Man erhält 0,47 g (90 % der Theorie) D,L-Serin mit einem Schmelzpunkt von 224-226 °C (Literatur : 228-236 °C).

Das D,L-Serin ist dünnschichtchromatographisch rein.

## Beispiel 6

1,56 g (0,005 Mol) 2-Azido-3-benzyloxy-propionsäure-benzylester werden in 10 ml absolutem Ethanol gelöst und mit 0,20 g Palladium auf Aktivkohle (10 %ig) versetzt. Diese Reaktionsmischung wird in einem Autoklaven 25 Stunden bei 40 bar Wasserstoffdruck und einer Reaktionstemperatur von 50 °C hydriert. Danach werden 5 ml Wasser und weitere 0,1 g Palladium/Aktivkohle zugegeben und anschliessend 24 Stunden bei 50 bar Wasserstoffdruck und einer Reaktionstemperatur von 70 °C hydriert.

Nach Filtration wird das Filtrat bis zur Trockne eingedampft und der feste Rückstand 24 Stunden bei 50 °C unter vermindertem Druck getrocknet. Man erhält 0,51 g (97 % der Theorie) D,L-Serin mit einem Schmelzpunkt von 229-231 °C (Literatur : 228-236 °C).

Das D,L-Serin ist dünnschichtchromatographisch rein.

## Beispiel 7

1,56 g (0,005 Mol) 2-Azido-3-benzyloxy-propionsäure-benzylester werden in 10 ml absolutem Methanol gelöst, 0,70 g (0,019 Mol) trockenes Salzsäuregas eingeleitet und mit 0,20 g Palladium auf Aktivkohle (10 %ig) versetzt. Diese Reaktionsmischung wird in einem Autoklaven 25 Stunden bei 25 °C und einem Wasserstoffdruck von 20 bar hydriert. Nach Filtration wird das Filtrat am Rotationsverdampfer eingeengt und der Rückstand aus Methanol/Diethylether (Volumenverhältnis etwa 1 : 1) umkristallisiert.

Man erhält 0,75 g (96 % der Theorie) D,L-Serin-methyl-ester-hydrochlorid mit einem Schmelzpunkt von 131-133 °C (Literatur : 131-134 °C).

### Beispiel 8

· 1,56 g (0,005 Mol) 2-Azido-3-benzyloxy-propionsäure-benzylester werden in 10 ml absolutem Ethanol gelöst, 1,10 g (0,03 Mol) trockenes Salzsäuregas eingeleitet und mit 0,20 g Palladium auf Aktivkohle (10 %ig) versetzt. Diese Reaktionsmischung wird in einem Autoklaven 40 Stunden bei 25 °C und einem Wasserstoffdruck von 20 bar hydriert. Nach Filtration wird das Filtrat am Rotationsverdampfer eingeengt und der Rückstand aus Ethanol/Diethylether (Volumenverhältnis etwa 1 : 1) umkristallisiert. Man erhält 0,81 g (96 % der Theorie) D,L-Serin-ethylester-hydrochlorid mit einem Schmelzpunkt von 102-103,5 °C (Literatur : 100-102 °C).

### Beispiel 9

1,56 g (0,005 Mol) 2-Azido-3-benzyloxy-propionsäure-benzylester werden in 10 ml Ethanol gelöst, 1,50 g (0,041 Mol) trockenes Salzsäuregas eingeleitet und 0,03 g Rhenium-(VII)-sulfid zugegeben. Diese Reaktionsmischung wird in einem Autoklaven 20 Stunden bei 25 °C und einem Wasserstoffdruck von 20 bar hydriert. Nach Abfiltrieren des Katalysators wird das Lösungsmittel abgedampft. Das erhaltene Öl (1,42 g = 88 % rohes O-Benzyl-D,L-Serin-benzylester-hydrochlorid) wird in Chloroform gelöst und mit Diethylether versetzt. Die ausgefallenen Kristalle werden dreimal aus Ethanol/Diethylether umkristallisiert. Man erhält 0,78 g (48 % der Theorie) O-Benzyl-D,L-Serinbenzylester-hydrochlorid mit einem Schmelzpunkt von 144,5-145,5 °C (Literatur : 147,5-148,5 °C).

### Beispiel 10

1,56 g (0,005 Mol) 2-Azido-3-benzyloxy-propionsäure-benzylester werden in 10 ml absolutem Ethanol gelöst und 0,20 g Palladium auf Aktivkohle (10 %ig) dazugegeben. In die stark gerührte Suspension wird 8 Stunden lang durch eine Fritte Wasserstoff eingeleitet. Danach wird abfiltriert, das Filtrat verworfen und der Filterrückstand mit heissem Wasser extrahiert. Der Extrakt wird am Rotationsverdampfer eingeengt, das ausgefallene Produkt abgetrennt und 24 Stunden lang bei 50 °C unter vermindertem Druck getrocknet.

Man erhält 0,77 g (79 % der Theorie) O-Benzyl-D,L-Serin mit einem Schmelzpunkt von 202-205 °C (Literatur : 218 °C).

### Beispiel 11

1,56 g (0,005 Mol) 2-Azido-3-benzyloxy-propionsäure-benzylester werden gemäss Beispiel 10, aber in einem Autoklaven bei 25 °C 20 Stunden lang unter 30 bar Wasserstoffdruck hydriert.

Nach Aufarbeitung erhält man 0,80 g (82 % der Theorie) O-Benzyl-D,L-Serin mit einem Schmelzpunkt von 205-207 °C.

### Ansprüche

1. 2-Azido-3-benzyloxy-propionsäure-benzylester.

2. Verfahren zur Herstellung des 2-Azido-3-benzyloxypropionsäure-benzylesters gemäss Anspruch 1, dadurch gekennzeichnet, dass man 2-Chlor-acrylnitril mit der zweifachen molaren Menge Benzylalkohol zum 3-Benzyloxy-2-chlor-propionsäure-iminobenzylester umsetzt, diesen sauer zum 3-Benzyloxy-2-chlor-propionsäure-benzylester verseift und schliesslich das Chloratom mittels eines Alkalimetallazids in Gegenwart eines Phasentransferkatalysators gegen eine Azidogruppe austauscht.

3. Verwendung des 2-Azido-3-benzyloxy-propionsäure-benzylesters gemäss Anspruch 1 als Zwischenprodukt bei der Herstellung von D,L-Serin oder Derivaten des D,L-Serins.

### Claims

1. 2-azido-3-benzyloxy-propionic acid-benzyl ester.

2. Process for the production of 2-azido-3-benzyloxy-propionic acid-benzyl ester according to claim 1, characterised in that 2-chloro-acrylonitrile is reacted with a double molar quantity of benzyl alcohol to produce 3-benzyloxy-2-chloro-propionic acid-iminobenzyl ester, which is saponified acidically to produce 3-benzyloxy-2-chloro-propionic acid-benzyl ester and then the chlorine atom is exchanged for an azido-group using an alkali metal azide in the presence of a phase transfer catalyst.

3. Use of 2-azido-3-benzyloxy-propionic acid-benzyl ester according to claim 1 as an intermediate in the production of D,L-serine or derivatives of D,L-serine.

**Revendications**

1. Benzylester de l'acide 2-azido-3-benzyloxypropionique.

2. Procédé pour la fabrication du benzylester de l'acide 2-azido-3-benzyloxy-propionique suivant la revendication 1, caractérisé en ce que l'on fait réagir du 2-chlor-acrylonitrile avec le double de la quantité molaire d'alcool benzylique pour former l'iminobenzylester de l'acide 3-benzyloxy-2-chloro-propionique, que l'on saponifie par un acide en benzylester de l'acide 3-benzyloxy-2-chloro-propionique, et finalement, échange l'atome de chlore contre un groupe azido, au moyen d'un azide de métal alcalin, en présence d'un catalyseur de transfert de phase.

3. Utilisation du benzylester de l'acide 2-azido-3-benzyloxy-propionique suivant la revendication 1, comme produit intermédiaire pour la fabrication de la D,L-sérine ou des dérivés de la D,L-sérine.